# EUROPEAN PATENT APPLICATION

(11) **EP 2 679 669 A1**
(43) Date of publication of application: **01.01.2014**
(21) Application number: 12004798.0
(22) Date of filing: 26.06.2012
(51) Int. Cl.: C12N 5/00, C12N 5/0775, A61L 27/52

(54) **Fabrication of three-dimensional hydrogel objects**

(71) Applicant: ETH Zurich, 8092 Zurich (CH)
(72) Inventor: Wüst, Silke, 8049 Zurich (CH); Schneider, Roman, 9205 Waldkirch (CH); Hofmann, Sandra, 3014 Bern (CH); Müller, Ralph, 8704 Herrliberg (CH)

(57) **Abstract**

A process for preparing three-dimensional hydrogel objects comprising living cells is disclosed. A cell suspension is provided, the suspension comprising a first hydrogel precursor, which exhibits a transition into a gel state by a reduction in temperature below the gel point, comprising a second hydrogel precursor, which is chemically cross-linkable by a non-cytotoxic cross-linking agent, and comprising living cells. The cell suspension is deposited onto a substrate by printing or plotting and cooled to a temperature below the gel point of the cell suspension to solidify the deposited cell suspension. Thereafter a cross-linking agent is added to the solidified cell suspension so as to induce chemical cross-linking of the second hydrogel precursor. In this process, initial structural stability is achieved rapidly by temperature-dependent gelation, while long-term stability is ensured by chemical cross linking. Also disclosed is a composition of matter comprising the first and second hydrogel precursor and living cells, and a device for carrying out the process.

## Description

### TECHNICAL FIELD

The present invention relates to a process, a composition of matter and a device in the area of biomedical engineering. More particularly, the present invention relates to a process for fabrication of three-dimensional hydrogel objects and to a composition of matter and device which may be used in such a process.

### PRIOR ART

Hydrogels have been widely used as scaffold material in tissue engineering especially for soft tissue applications. Many hydrogels are biocompatible and if necessary, cells can be included. For example, it is known to pour a hydrogel precursor into a pre-shaped mold. The mold can be, for example, a disc shape in vitro. It is also possible to fill an incision in a certain tissue in vivo.

Lately 3D bioprinting started penetrating the field of tissue engineering. Hydrogels are especially suitable for bioprinting approaches since they can be printed in their liquid state and can be solidified right after printing. Nevertheless, still methods are lacking of how to control the solidification process in order to guarantee a predetermined, precise and stable scaffold geometry.

Alginate-hydroxyapatite hydrogels have been successfully used for tissue engineering of bone. Nevertheless the hydrogel system has some major limitations especially concerning its application for bioprinting and its structural stability. Direct printing of alginate-hydroxyapatite hydrogels into a cross-linker solution produces stable single layers, but there is no binding between additional layers, which limits its upscaling to clinically applicable 3D dimensions. In addition, cells which have been printed in the first layers of the construct are exposed longer to the cross-linker than the cell in the last layers. On the other hand, if the whole construct is cross-linked only after printing, blurring of the material takes place which results in an inaccurate shape of the construct.

The dissertation of André Heeg, "Bioplotting mit vitalen Zellen", University of Freiburg im Breisgau, (2010) discloses a hydrogel plotting process that uses alginate as a sole hydrogel precursor. The structural stability of plotted alginate is guaranteed by instantaneous freezing with liquid nitrogen to a temperature between -5 °C and -16 °C. After printing the construct is thawed and chemically cross-linked with CaCl₂ solution. While the process has the potential for obtaining constructs exhibiting a stable shape, the freezing process can lead to cell death due to ice development.

Kentaro Iwami et al., "Bio rapid prototyping by extruding/aspirating/refilling thermoreversible hydrogel", Biofabrication 2 (2010) 014108, discloses the use of a thermoreversible hydrogel, Mebiol gel (N-isopropylamid and poly oxyethylene), for rapid prototyping. Mebiol gel is fluid below 15 °C and its sol-gel transition temperature is at 22 °C. The gel solution in the nozzle was kept below 10 °C maintaining its sol state and extruded onto a substrate in a 30 °C environment, which led to gelation of the hydrogel precursor. A disadvantage of this approach is that the hydrogel is extruded into an environment at elevated temperatures. The construct must be kept at an elevated temperature after extrusion in order to maintain its dimensional stability, which poses constraints on the handling procedures after printing to avoid cellular stress.

Natalja Fedorovich et al., "Distinct tissue formation by heterogeneous printing of osteo-and endothelial progenitor cells", Tissue Engineering Part A, 17(15-16) (2011) 2113-21, discloses a printed two-phase construct consisting of layers of Matrigel and separate layers of alginate. Matrigel preparations were processed at 4 °C to keep the matrix fluid, and formed a gel at room temperature during printing. Alginate was incubated in CaCl₂ after printing for cross-linking. Both hydrogels contained living cells. This approach cannot overcome the limitations of the approaches discussed above. In particular, in this approach the alginate layers lack structural stability before cross-linking, and the fact that the Matrigel layers must be kept at a relatively high temperatures after printing leads to handling restrictions of the resulting construct and possible cellular stress for the embedded cells.

### SUMMARY OF THE INVENTION

It is an object of the present invention to provide a process for fabricating three-dimensional hydrogel objects which allows instant solidification of the hydrogel upon printing or plotting without harming incorporated cells, while ensuring that the final object will have a stable shape.

This object is achieved through a process as defined in claim 1.

It is another object of the present invention to provide a composition of matter which is specifically adapted to be used in such a process. This object is achieved by a composition as defined in claim 8.

It is yet another object of the present invention to provide an apparatus which is specifically adapted for carrying out such a process. This object is achieved by an apparatus as defined in claim 14.

Preferred embodiments are specified in the dependent claims.

The present invention provides a process for preparing three-dimensional hydrogel objects comprising living cells, the process comprising:
(a) providing a fluid cell suspension exhibiting a transition into a gel state by a reduction in temperature below its gel point, the cell suspension comprising a first hydrogel precursor exhibiting a transition into a gel state by a reduction in temperature, comprising a second hydrogel precursor, which is chemically cross-linkable by a non-cytotoxic cross-linking agent, and comprising living cells;
(b) depositing the cell suspension onto a substrate by printing or plotting;
(c) cooling the deposited cell suspension to a temperature below its gel point to solidify the deposited cell suspension; and
(d) adding a cross-linking agent to the solidified cell suspension so as to induce chemical cross-linking of the second hydrogel precursor.

Before the printing or plotting step, the cell suspension should be kept at or brought to a temperature above its gel point in order to maintain a fluid state of the cell suspension. During and/or after the printing or plotting step, the cell suspension is cooled to a temperature below its gel point in order to rapidly solidify the deposited cell suspension. The cell suspension is then kept at a temperature below its gel point at least until chemical cross-linking of the second hydrogel precursor has taken place.

The gel point is the critical temperature below which the cell suspension turns into its gel state. At the gel point, a sudden increase in viscosity is observed, which can be determined by rheology measurements. In particular, the gel point can be defined as the temperature at which the shear storage modulus G' and shear loss modulus G", as determined by standard dynamic mechanical analysis methods, intersect upon cooling. By the way of example, the gel point can be determined by oscillatory measurements in temperature ramp oscillation mode with constant shear stress amplitude of 50 Pa, a frequency of 1 Hz, and a temperature ramp of 1 °C/min. The gel point of the cell suspension will depend on the properties and concentration of the first hydrogel precursor, which is primarily responsible for the behavior that the cell suspension as a whole solidifies when cooled below a certain temperature, and will further depend on the other components of the cell suspension, in particular, on the second hydrogel precursor and possibly on the kind and amount of additives, if any.

In the process, a 3D object is created by depositing the cell suspension onto the substrate. This can be done for example layer-by-layer, i.e., after printing or plotting a first layer of cell suspension, additional hydrogel layers are printed or plotted on top of the first layer to generate a three-dimensional object. By cooling the cell suspension below the gel point, the printed or plotted construct is initially dimensionally stabilized. This initial stabilization should preferably be done during and/or immediately after printing or plotting, preferably as soon as the material touches the substrate. Only after such initial stabilization, the solidified cell suspension is further treated by a cross-linking agent, which induces chemical cross-linking of the second hydrogel precursor, to permanently stabilize the construct. Preferably, this treatment is carried out only after the entire 3D object has been constructed. However, it is also conceivable that the cross-linking agent is added earlier, in principle even already after printing or plotting one or more voxels or one or more layers of the cell suspension, even though this is generally not preferred. After cross-linking of the second hydrogel precursor has taken place, the resulting object can be handled at temperatures even above the gel point of the cell suspension without losing its structural stability.

The proposed process renders a more accurate 3D structure than previously proposed methods. Initial stability is provided by solidifying the printed voxels or the plotted paths instantaneously after printing or plotting, keeping their shape. This is possible due to the temperature-dependent behavior of the first hydrogel precursor. The second hydrogel precursor only solidifies when it gets into contact with a cross-linking agent. This chemical process is slower than the temperature-dependent physical solidification, typically requiring several minutes in many known systems. Chemical cross-linking should be carried out before heating up the material again above the gel point of the cell suspension, so as to guarantee the stability of the geometry, in particular, before placing the object in an incubator.

In a simple, yet effective, embodiment, the cell suspension is cooled after its deposition by keeping the substrate below the gel point. In this manner, the cell suspension is automatically cooled once it reaches the substrate or the material which has been deposited on the substrate earlier. The substrate may be kept at the desired temperature in a variety of different ways, e.g., by placing a cold reservoir such as a commercially available coldpack below the substrate. In more sophisticated embodiments, the substrate may be cooled by an active cooling device such as a Peltier element, or may be cooled by a heat exchanger through which a cold medium is pumped. However, it is also conceivable to achieve cooling of the cell suspension after printing or plotting in other known ways, e.g., by directing a stream of cold gas such as cold nitrogen gas at the cell suspension.

Before the cell suspension is deposited onto the substrate, the cell suspension should be brought to a temperature above the gel point, so as to liquefy the cell suspension. Since for many systems the gel point will be above ambient temperature (i.e., above 20-25 °C), this may involve actively heating the cell suspension to a temperature above the gel point. The cell solution is preferably heated up to a maximum of no more than 37-40 °C to ensure cell survival. Temperatures may vary depending on the cell type and first hydrogel precursor.

The process may further comprise eluting the first hydrogel precursor after chemical cross-linking of the second hydrogel precursor to create a porous three dimensional hydrogel object comprising living cells. This is preferably achieved by incubating the three-dimensional hydrogel object in cell culture medium under cell specific conditions, preferably at or around 37 °C, until at least a portion of the first hydrogel precursor has been washed out, to finally obtain a porous three-dimensional hydrogel object. Alternatively, the object may be incubated in any other cell compatible liquid until at least a portion of the first hydrogel precursor is eluted. The incubation temperature may vary depending on the cell type.

Generally speaking, the gel point should preferably be in a range such that the system can be cooled to a temperature that is at least 10 °C lower that the gel point without affecting cell viability. Cooling to a temperature at least 10 °C below the gel point ensures instantaneous gelation. Preferably the first hydrogel precursor is selected to have a gel point between approximately 10 °C and approximately 30 °C, preferably between 15 °C and 25 °C. Favorably a temperature well above 0 °C is chosen during the cooling process so as not to affect cell viability due to intracellular ice formation.

In particular, the first hydrogel precursor may be chosen to be at least one of gelatin and a mixture of pectin and chitosan. The gel point for gelatin is between 0 and 35 °C, depending on gelatin grade and concentration. For an aqueous 5% gelatin solution the gel point is around 20 °C. In a mixture of pectin and chitosan, the gel point depends on the pectin content. For a 30%/70% (w/w) pectin-chitosan mixture, the gel point is at around 37 °C; for lower pectin content, the gel point will be lower. Therefore, it is preferred to use a pectin content of less than approximately 30% (w/w). In order to ensure thermoreversible gelation, the pectin content should be above approximately 20% (w/w).

The second hydrogel precursor may be chosen to be at least one of alginate, cellulose derivatives such as sodium carboxymethylcellulose or hydroxyethyl cellulose, chitosan, chondroitin sulphate, chondroitin sulfate-PEG, hyaluronic acid, pectin gel, poly(L-lactide), poly(vinyl alcohol), xanthan, and fibrin. Alginate may be chemically cross-linked by addition of calcium ions such as by addition of a solution of CaCl₂, CaCO₃, and/or CaSO₄. Cross-linkers for alternative gelation agents are listed in the table below:

| **Gelation agent** | **Cross-linker** |
|---|---|
| Alginate | Calcium ions (e.g., CaCl₂, CaCO₃, CaSO₄) |
| Hyaluronic acid | Carbodiimide |
| | PEG |
| | Adipic dihydrazide |
| Cellulose derivatives: sodium carboxymethylcellulose, hydroxyethyl cellulose | Divynilsulphone |
| Chitosan | Sodium hydroxide, Ethanol |
| Chondroitin sulphate | Diaminododecane |
| Chondroitin sulfate-PEG | PEG-(NH₂)₆ |
| Pectin Gel | Calcium ions or divinylsulfone |
| Poly(L-lactide) | Macromer with a few PHEMA (poly hydroxyethyl methacrylate) groups incorporated |
| Poly(vinyl alcohol) | Aldehyde |
| Xanthan | Epichlorohydrin |
| Fibrin | Enzyme-catalyzed cross-linking of fibrinogen fibrinolytic inhibitors |

The concentration of cells may be chosen according to the intended use of the object and may be, e.g. between 0.1 and 20 million cells, preferably between 0.5 and 8 million cells per mL of precursor mixture. Cells of a single cell type or multiple cell types may be suspended into the precursor mixture depending on the tissue of interest. The cells may optionally be embedded in microcapsules.

The present invention further provides a corresponding composition for preparing three-dimensional hydrogel objects, comprising:
a first hydrogel precursor, which exhibits a transition into a gel state by a reduction in temperature below a gel point;
a second hydrogel precursor, which is chemically cross-linkable by a non-cytotoxic cross-linking agent; and
living cells.

The composition will generally take the form of a suspension.

Additional materials may optionally be added to the composition to confer further properties to the three-dimensional object. In this regard, additional materials may influence printability, biocompatibility, bioactivity, object stiffness, mechanical strength, mechanical integrity, pore size, porosity, degradability, cell attachment, cell stimuli in terms of cell viability, cell proliferation or cell differentiation, pH, hydrophilicity, solute transport, change of gel point (towards lower or higher temperature). In particular, the composition may further comprise an X-ray opaque material to confer X-ray opaqueness to the material. With regard to X-ray opaqueness, at least one of hydroxyapatite (HA), calcium phosphate and other calcium composites, magnesium phosphate and other magnesium composites, titanium oxide, zinc oxide, zirconium oxide, silicon, biocompatible metal compositions, biocompatible ceramic compositions may be included as a contrast agent for e.g. computer tomographic imaging. By the way of example, between 0.01 and 0.15 g HA, preferably between 0.05 and 0.10 g HA, especially around 0.08 g HA may be added to 1 mL of precursor mixture to obtain ideal hydrogel object stiffness and X-ray opaqueness properties. It is further imaginable to use other biocompatible contrast agents for alternative imaging methods.

The invention further provides an apparatus for preparing three-dimensional hydrogel objects comprising living cells, the apparatus comprising:
(a) a discharge device for discharging a cell suspension onto a substrate;
(b) a heating device in contact with said discharge device for heating the cell suspension before discharge;
(c) an X-Y positioning platform for positioning the discharge device and the substrate relative to each other in a predetermined plane;
(d) a cooling device for cooling the cell suspension after discharge; and
(e) a Z positioning platform for positioning the discharge device and the substrate relative to each other in a direction perpendicular to the substrate.

The discharge device may, e.g., comprise a syringe for use as a cell suspension container. The cell solution is then extruded preferably by a controllable, motor-driven syringe plunger. Alternatively, an ink-jet print head may be used as a discharge device. The construction of ink-jet print heads is generally well known in the art.

The heating device may heat a container containing the cell suspension. Alternatively or additionally, it may heat only a nozzle portion of the discharge device. If a syringe is used as a container, the syringe is advantageously positioned within a heating arrangement encircling the syringe for heating and liquefying the cell suspension. In particular, the heating arrangement may comprise a solid metal block having a bore in which the syringe is placed, the metal block being in thermal contact with a heater such as a Peltier element, a thick- or thin-film heater, a heat exchanger etc. In addition, the syringe outlet may be surrounded by a second heating arrangement. One or more temperature sensors and a controller receiving temperature signals from the sensors may be provided to control the heating device.

During the printing or plotting process, an X-Y positioning platform controls the X-Y position of the discharge device relative to the substrate in a predetermined plane (which will be the plane defined by the substrate in the case of a planar substrate). This plane will in the following be called the horizontal plane. To this end, either the discharge device or the substrate may be mounted to the X-Y positioning platform, while the other part is stationary. Suitable X-Y positioning platforms are well known in the art.

The printed or plotted cell suspension is cooled by a cooling device. In particular, the cooling device may be in thermal contact with the substrate so as to cool the substrate. The cooling device may be, e.g., a Peltier element, a heat exchanger or a flat coldpack.

A controllable Z positioning platform beneath the cooling element controls the distance between the discharge device and the substrate so as to enable deposition of the cell suspension onto the substrate. The Z positioning platform may be either connected to the discharge device or to the substrate. The Z direction will in the following also be called the vertical direction.

An external control unit, such as an appropriately programmed general-purpose computer with suitable driving circuits, may control the X-Y positioning platform and the Z positioning platform so as to coordinate the process of preparation of three-dimensional hydrogel objects comprising living cells. To this end, the control unit may be adapted to receive CAD/CAM data of the object to be prepared.

### BRIEF DESCRIPTION OF THE DRAWINGS

Preferred embodiments of the invention are described in the following on the basis of the figures which merely serves as an explanation and are not interpreted to be restrictive. The figures show:

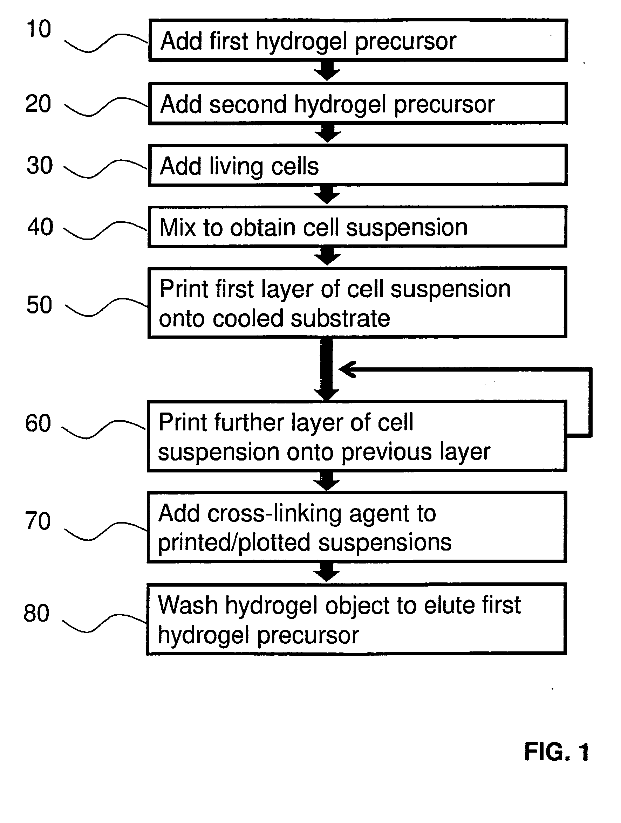

- Fig. 1: shows a process flowchart illustrating the steps of an illustrative process for fabricating three-dimensional hydrogel objects in accordance with the principles of the invention;

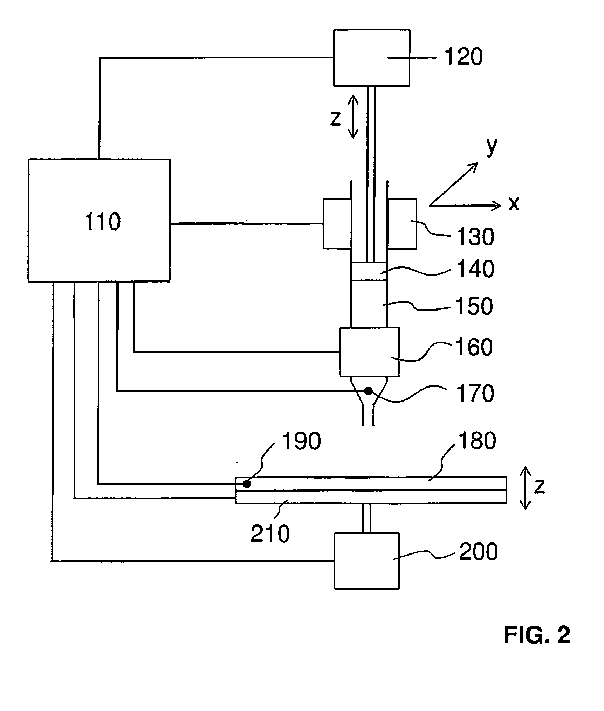

- Fig. 2: shows, in a highly schematic fashion, the setup of a 3D printer which may be used in conjunction with the present invention;

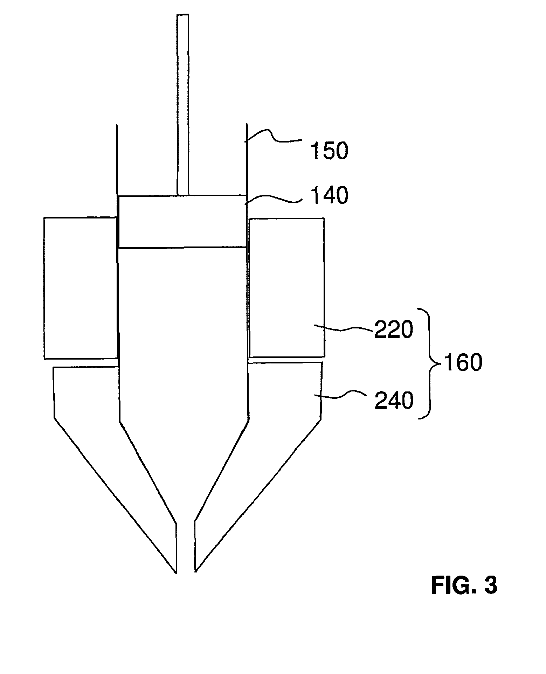

- Fig. 3: shows, in a highly schematic fashion, a heating arrangement for a syringe which may be used in conjunction with the present invention;

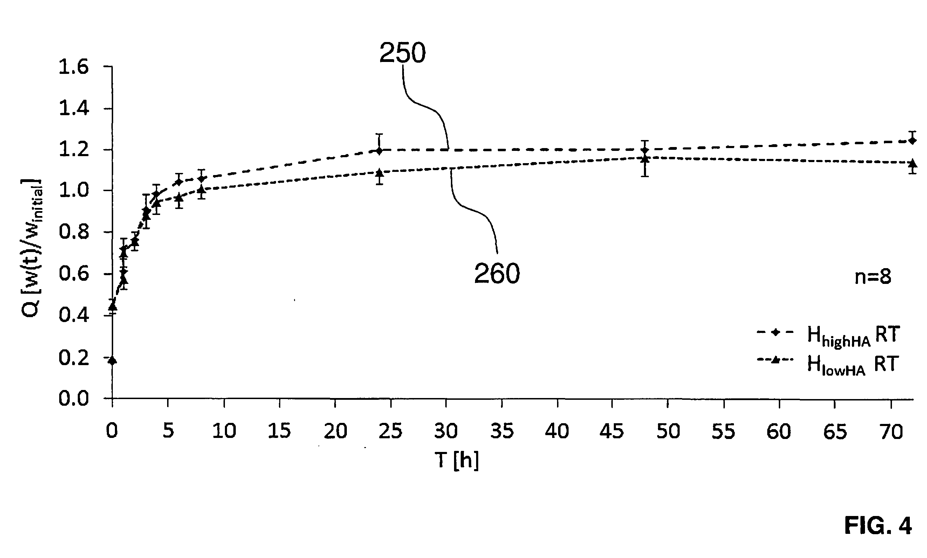

- Fig. 4: shows swelling data of alginate-gelatin-HA hydrogel objects at room temperature (RT) during 72 hours (h) by measuring the hydrogel weight over time. Hydrogel disks (n=8 per group) (H_{highHA} 250 and H_{lowHA} 260) with diameter of 5 mm and 2 mm height were prepared by pouring the solution into a cooled mold, cross-linked for 10 minutes in PBS with 2% (w/v) CaCl₂, weighed and dried. Dried samples were immersed in phosphate buffered saline (PBS) with 0.25% (w/v) CaCl₂ enhanced with 100 mM sodium azide and weighed over time. Hydrogel swelling is reported as ratio of hydrogel weight over time (72h) compared to the initial hydrogel weight measured directly after preparation: (Q = w(t)/w_{Initial});

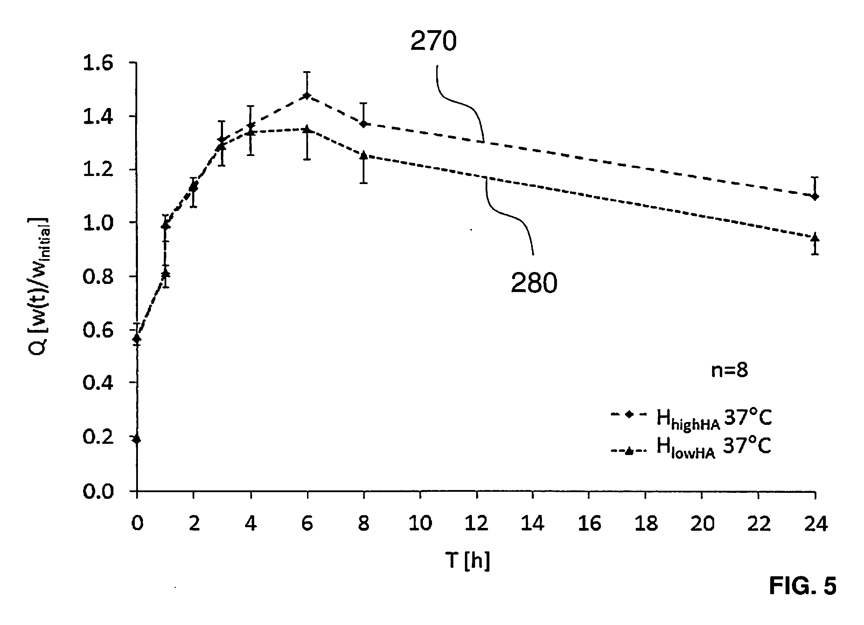

- Fig. 5: shows degradation data of alginate-gelatin-HA hydrogel objects at 37 °C after immersion of dried hydrogel disks in solution reported as ratio of hydrogel weight over time compared to the initial hydrogel weight. In Figure 5 the first 24 hours of degradation are shown. The maximum hydrogel swelling is reached after 6h with an additional swelling of up to 50% (H_{highHA} 270: 48 ± 9 % at 6h, H_{lowHA} 280: 35 ± 12 % at 6h). Beyond this time point degradation takes place;

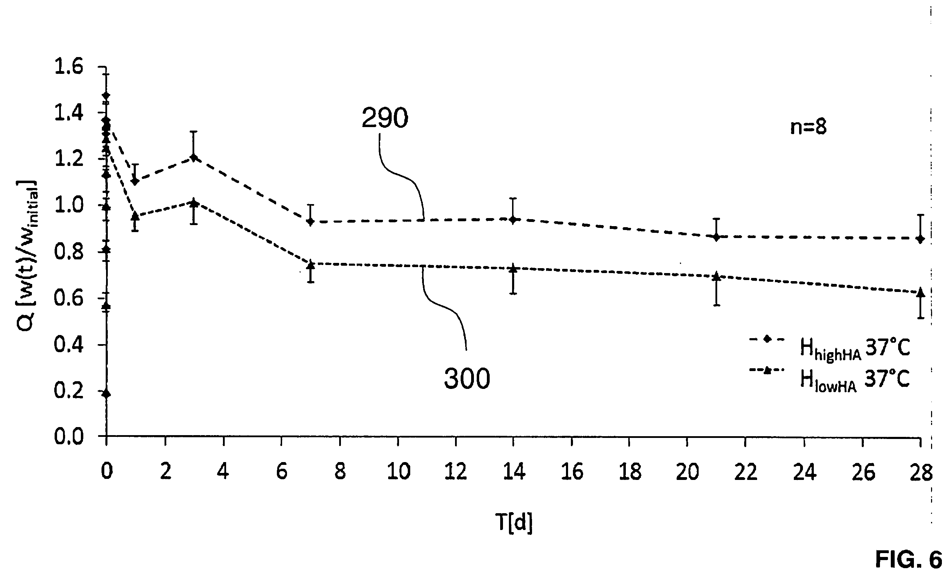

- Fig. 6: shows degradation data of alginate-gelatin-HA hydrogel objects at 37 °C after immersion of dried hydrogel disks in solution reported as ratio of hydrogel weight over time compared to the initial hydrogel weight. In Figure 6 the first 28 days of degradation are shown, which show a loss of hydrogel weight of less than 40% after 4 weeks (H_{highHA} 290: 78 ± 10 % after 4 weeks, H_{lowHA} 300: 64 ± 12 % after 4 weeks);

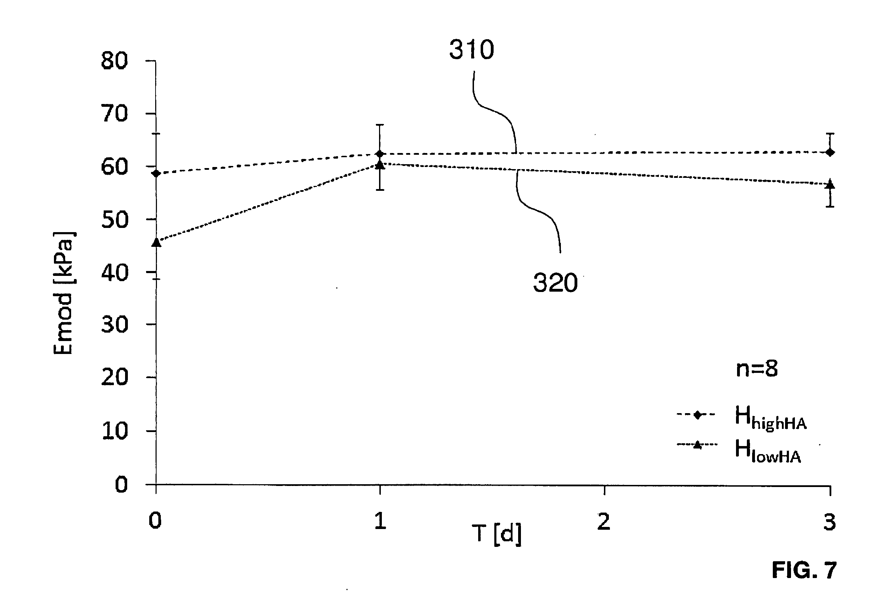

- Fig. 7: shows mechanical properties of alginate-gelatin-HA hydrogel objects. Unconfined uniaxial compression tests of bulk material were performed at room temperature (n=8 per group). Tests were done position-controlled with a pre-load of 5 mN and samples were compressed with a speed of 5mm/min until 60% deformation (ε=60%). The E-modulus (Emod) was determined at the linear region of the stress-strain curve at strains between ε=5-15%. Figure 7 shows the E-modulus at day 0, day 1 and day 3 of H_{highHA} 310 and H_{lowHA} 320. The E-modulus varies over time with the two materials behaving statistically significantly different over time;

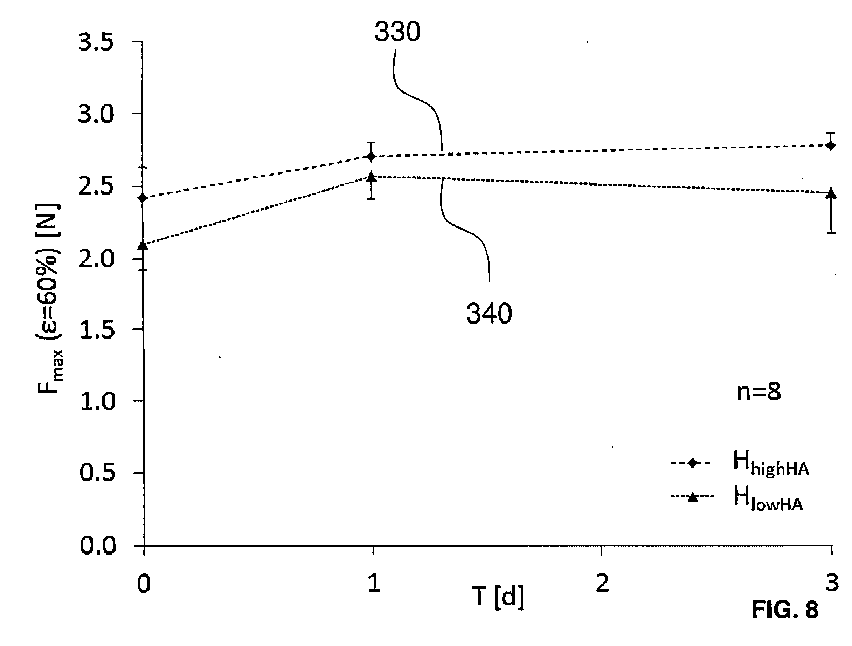

- Fig. 8: shows mechanical properties of alginate-gelatin-HA hydrogel objects. Unconfined uniaxial compression tests of bulk material were performed at room temperature (n=8 per group). Tests were done position-controlled with a pre-load of 5 mN and samples were compressed with a speed of 5mm/min until 60% deformation (ε=60%). Figure 8 shows the maximal force Fₘₐₓ at 60% stress at day 0, day 1 and day 3. The hydrogel H_{highHA} 330 with a higher HA content results in higher stiffness than H_{lowHA} 340. The maximal force vary over time, with no statistically significant difference in material behavior over time;

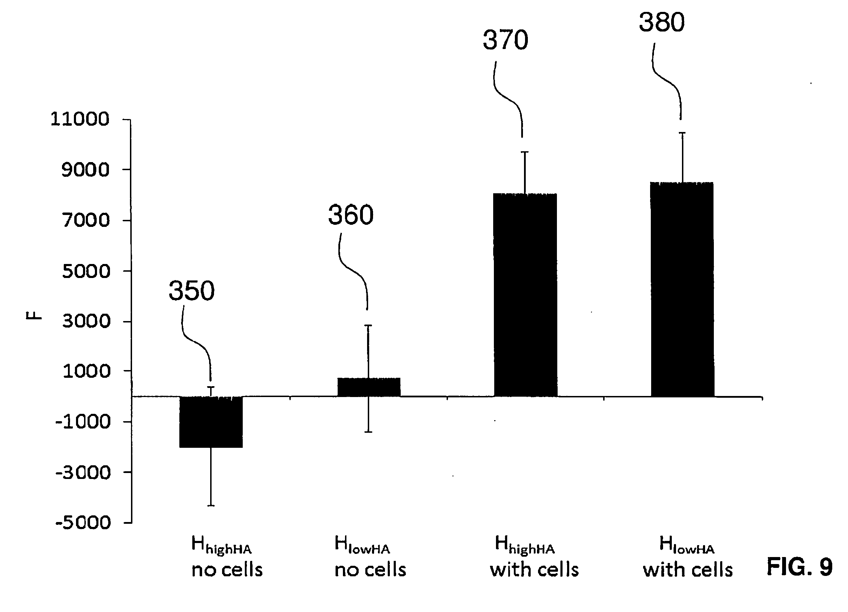

- Fig. 9: shows cell metabolic activity of alginate-gelatin-HA hydrogel objects. Human mesenchymal stem cells were mixed into the hydrogel precursor mixture at 37 °C at a density of 1 million cells/mL before printing. Samples were printed on a cooled substrate, cross-linked for 10 minutes in PBS with 2% (w/v) CaCl₂ and cultured in control medium enhanced with 0.25% (w/v) CaCl₂ at 37 °C. Cell metabolic activity was measured by measuring fluorescence (F) normalized to hydrogel weight using an alamarBlue^{®} assay 24 hours after printing. H_{highHA} 350 and H_{lowHA} 360 without cells were compared against H_{highHA} 370 and H_{lowHA} 380 with cells;

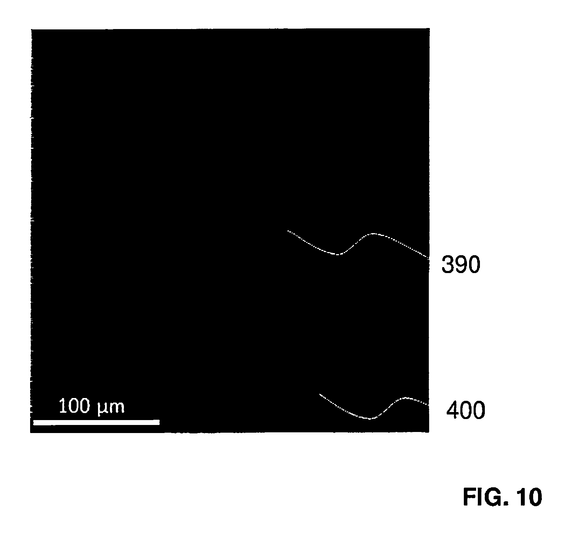

- Fig. 10: shows a LIVE-DEAD assay of an alginate-gelatin-HA hydrogel object. A representative sample of the group supplemented with 4 g HA is shown. The assay was performed on samples after three days subsequent to printing. The LIVE-DEAD viability-cytotoxicity assay provides a two-signal fluorescence image that is based on the simultaneous determination of living 390 and dead 400 cells. For both HA materials living cells could be detected with the fluorescence microscope.

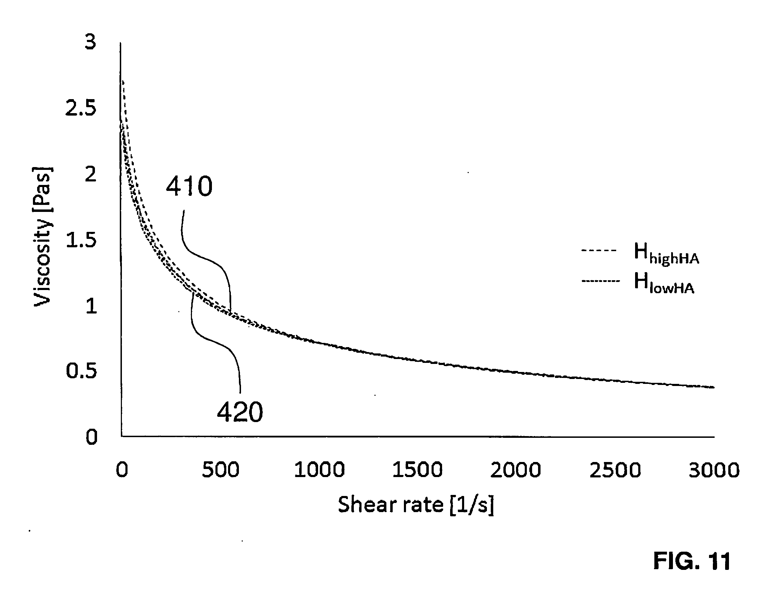

- Fig. 11: shows the viscosity of an alginate-gelatin-HA hydrogel object. Rheological properties were based on rotatory measurements. The measurement was performed using a rheometer with a cone-plate measuring geometry (diameter 35mm, cone to plate distance 0.105 mm, cone angle 2°). A sample of 425-430 mg hydrogel (400 µl) was placed in the gap between the rotating cone and the stationary flat plate. Rotatory tests were performed to obtain viscosity dependent of shear rate, which ranged between 0.1 and 3000 s⁻¹. The time duration for the whole measurement was set 800 s (400 seconds per way). A representative sample of H_{highHA} 410 and H_{lowHA} 420 of viscosity dependent on the shear rate is shown. Both hydrogels, H_{highHA} and H_{lowHA}, reveal the same behavior of viscosity dependent on the shear rate.

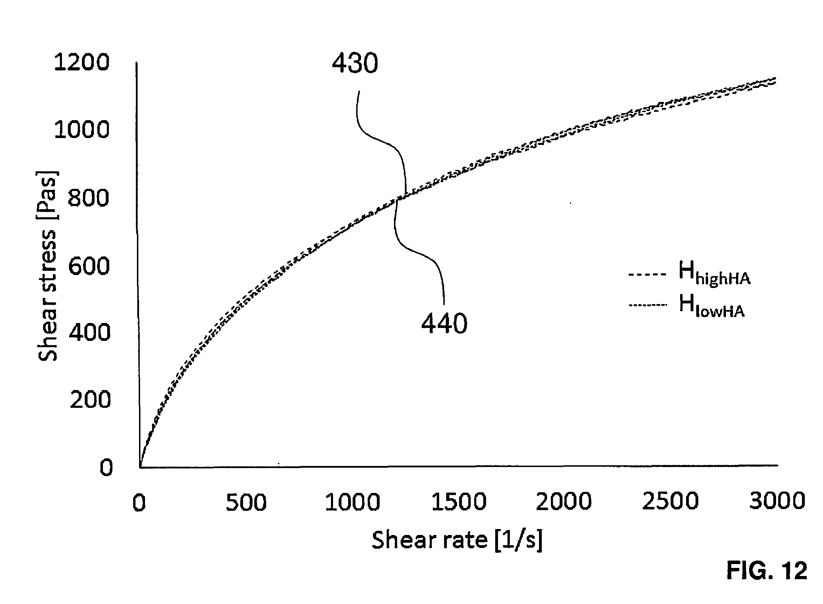

- Fig. 12: shows the shear stress of an alginate-gelatin-HA hydrogel object. Rheological properties were based on rotatory measurements. The measurement was performed using a rheometer with a cone-plate measuring geometry (diameter 35mm, cone to plate distance 0.105 mm, cone angle 2°). A sample of 425-430 mg hydrogel (400 µl) was placed in the gap between the rotating cone and the stationary flat plate. Rotatory tests were performed to obtain shear stress dependent of shear rate, which ranged between 0.1 and 3000 s⁻¹. The time duration for the whole measurement was set 800 s (400 seconds per way). A representative sample of H_{highHA} 430 and H_{lowHA} 440 of shear stress dependent on the shear rate is shown. Both hydrogels, H_{highHA} and H_{lowHA}, reveal the same behavior of shear stress dependent on the shear rate.

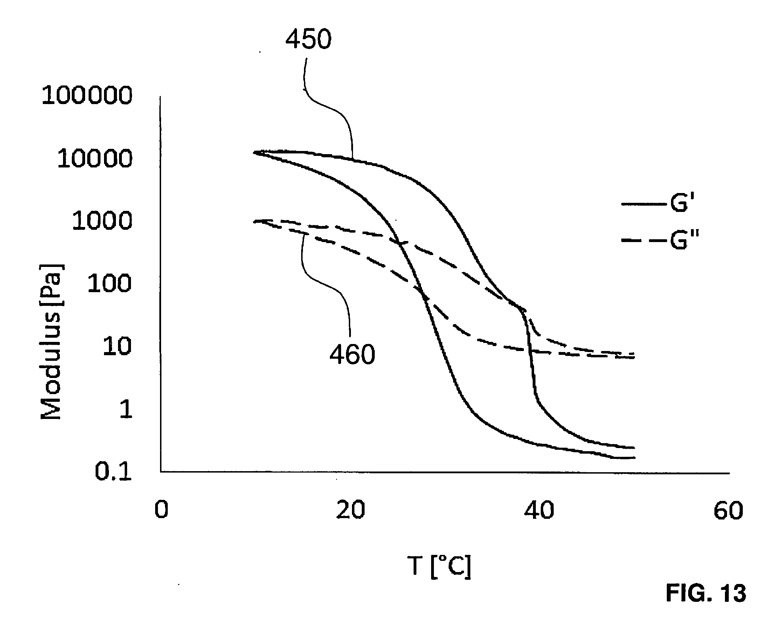

- Fig. 13: shows the gel point of an alginate-gelatin-HA hydrogel object. Rheological properties were based on oscillatory measurements. For a representative H_{highHA} sample the temperature (T) dependence of the storage modulus (G' 450) and loss modulus (G" 460) were measured using the temperature ramp oscillation mode with a constant shear stress amplitude of 50 Pa, a frequency of 1 Hz, and a temperature ramp of 1 °C/min. The temperature was set to 50 °C at the beginning of the test. During 80 min the sample was cooled down to 10 °C and reheated to 50 °C. The intersection of the storage modulus G' 450 and the loss modulus G" 460 during the cooling phase from 50 °C to 10 °C demonstrates the gel point. It is an estimation of the gelation temperature of a thermosensitive material. The average gel point for H_{highHA} is: 28.3 ± 0.6 °C (n=3).

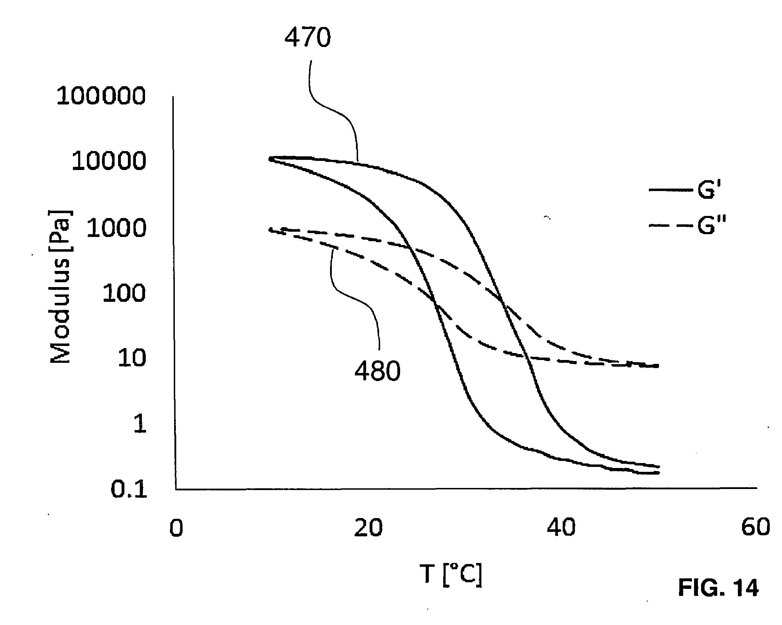

- Fig. 14: shows the gel point of an alginate-gelatin-HA hydrogel object. Rheological properties were based on oscillatory measurements. For a representative H_{lowHA} sample the temperature (T) dependence of the storage modulus (G' 470) and loss modulus (G" 480) were measured using the temperature ramp oscillation mode with a constant shear stress amplitude of 50 Pa, a frequency of 1 Hz, and a temperature ramp of 1 °C/min. The temperature was set to 50 °C at the beginning of the test. During 80 min the sample was cooled down to 10 °C and reheated to 50 °C. The intersection of the storage modulus G' 470 and the loss modulus G" 480 during the cooling phase from 50 °C to 10 °C demonstrates the gel point. It is an estimation of the gelation temperature of a thermosensitive material. The average gel point for H_{lowHA} is: 27.3 ± 0.3 °C (n=3).

### DESCRIPTION OF PREFERRED EMBODIMENTS

### Example 1

Three-dimensional hydrogel objects were prepared by carrying out the following process, as illustrated in Fig. 1:
In a first process step 10, 12.5% (w/v) of a first hydrogel precursor, here gelatin in water, which is thermally cross-linkable by a reduction in temperature, was added to a mixing device.
In a second process step 20, 2.5% (w/v) of a second hydrogel precursor, here alginate in water, which is chemically cross-linkable by a non-cytotoxic cross-linking agent, was added to the mixing device.
In a third process step 30, 1 million living cells/mL precursor mixture were added to the mixture in the mixing device.
In a fourth process step 40, the hydrogels and the cells were mixed at 37 °C to obtain a cell suspension.
In a fifth process step 50, a first layer of the cell suspension was printed onto a cooled substrate to instantly solidify the cell suspension immediately subsequent to printing. Since the gel point of the cell suspension comprising the first hydrogel precursor (gelatin) was approximately 27 °C, since the solidification process should be accelerated, and since any ice formation should be avoided, the substrate was cooled to a temperature between 0 and 10 °C.
In a sixth process step 60, a further layer of the cell suspension was printed on top of the solidified, cooled printed first layer. Since the first layer was cooled from below, this resulted again in instant solidification of the cell suspension of the second layer subsequent to printing. This step was repeated several times to obtain a layered 3D object.
In a seventh process step 70, the 3D object was immersed in an aqueous 2% (w/v) CaCl₂ solution as a cross-linking agent for the second hydrogel precursor (alginate). The hydrogel object was kept in the CaCl₂ solution for 10 min at 10 °C. This resulted in a stable three-dimensional hydrogel object, which afterwards could be handled at physiological temperatures without loss of dimensional stability.
In an eighth process step 80, the first hydrogel precursor was at least partially eluted at approximately 37 °C by incubation of the hydrogel object in a medium supplemented with 0.25% (w/v) CaCl₂, to create a porous three-dimensional hydrogel object.

### Example 2

In further examples, the alginate-gelatin-cell mixture was supplemented, before the mixing step 40, with 5% (w/v) hydroxyapatite (HA) for obtaining hydrogel objects with low HA content, and with 10% (w/v) HA for obtaining objects with high HA content.

### 3D printing device

Fig. 2 shows, in a highly schematic fashion, the setup of a 3D printer which may be used in conjunction with the present invention. A control unit 110 controls the printing/plotting process of the hydrogel objects. The hydrogel cell suspension is filled into a syringe 150 which is held on an X-Y positioning platform 130 and which is surrounded by a heating unit 160. A temperature sensor 170 at the outlet of the syringe measures the actual temperature of the cell suspension. The control unit 110 receives temperature signals from the temperature sensor 170 and controls the heating unit 160 to achieve a predetermined temperature of the cell suspension in the syringe. A motor drive 120 pushes a plunger 140 downwards in the syringe for extruding the cell suspension during the printing/plotting process. A cooling unit 210 mounted on a Z positioning platform 200 carries and cools a substrate 180. A second temperature sensor 190 measures the actual temperature of the substrate 180. The cooling unit 210 may be, e.g., a Peltier element. Alternatively, the cooling unit 210 may be represented by an coldpack.

Fig. 3 shows, in a highly schematic fashion, an advantageous heating device for a syringe which may be used in conjunction with the present invention. The syringe 150 is surrounded by a first heating unit 220. The heating unit 220 comprises a massive block of metal, e.g., aluminum, having a bore which receives the syringe body. The metal block is in thermal contact with a band heater. By using a massive metal block, optimal heat flow is 5 ensured, such that the cell suspension in the syringe is heated very homogeneously. A second heating unit 240 is provided at the outlet nozzle of the syringe 150 and the (syringe) tip, if any, so as to ensure that the cell suspension leaves the nozzle/tip at a well-defined temperature.

### Swelling and degradation properties

Fig. 4 shows swelling properties of alginate-gelatin-RA hydrogel objects fabricated according to Example 2. Swelling of the alginate-gelatin-HA hydrogel composition has been investigated at room temperature (RT) during 72 hours (h) by measuring the hydrogel weight over time. Hydrogel disks (n=8 per group) with diameter of 5 mm and 2 mm height were prepared, weighed and dried. Dried samples were immersed in phosphate buffered saline (PBS) with 0.25% (w/v) CaCl₂ enhanced with 100 mM sodium azide and weighed over time. Hydrogel swelling is reported as ratio of hydrogel weight over time compared to the initial hydrogel weight measured directly after preparation: (Q= w(t)/w_{Initial}). The graph shows that after 24 hours, equilibrium was reached. Regardless of the composition, hydrogels show a slight swelling of less than 30% of their original weight (H_{highHA} 250: 25 ± 5 % at 72h, H_{lowHA} 260: 15 ± 6 % at 72h).

Figs. 5 and 6 show degradation properties of the fabricated alginate-gelatin-HA hydrogel objects according to Example 2. For this study a time span of 4 weeks at 37 °C has been chosen to mimic hydrogel culture conditions resembling future tissue engineering culture conditions to a high degree. Medium was exchanged three times a week. Gelatin is expected to dissolve at 37 °C. Hydrogel disks (n=8 per group) with a diameter of 5 mm and 2 mm height were prepared, weighed and dried. Dried samples were immersed in PBS with 0.25% (w/v) CaCl₂ enhanced with 100 mM sodium azide. Hydrogel degradation is reported as the ratio of hydrogel weight over time compared to the initial hydrogel weight measured directly after preparation (Q= w(t)/wᵢₙᵢₜᵢₐₗ). Figure 5 shows the first 24 h of incubation. The maximum hydrogel swelling is reached after 6 h with an additional swelling of up to 50% (H_{highHA} 270: 48 ± 9 % at 6h, H_{lowHA} 280: 35 ± 12 % at 6h). From this time point onwards degradation takes place. Figure 6 shows the degradation over 4 weeks with a loss of hydrogel weight of less than 40% after 4 weeks (H_{highHA} 290: 78 ± 10 % after 4 weeks, H_{lowHA} 300: 64 ± 12 % after 4 weeks).

### Mechanical properties

Figs. 7 and 8 show mechanical properties of the alginate-gelatin-HA hydrogel objects fabricated according to Example 2. Unconfined uniaxial compression tests of bulk material were performed at room temperature (n=8 per group). Tests were done position-controlled with a pre-load of 5 mN and samples were compressed with a speed of 5 mm/min until 60% deformation (ε=60%). The E-modulus was determined at the linear region of the stress-strain curve at strains between ε=5-15%. Furthermore stress at ε=60% was determined. From the swelling and degradation experiment it is known that different time points reveal different properties of the material. In order to capture the change of material properties over time, the non-destructive mechanical tests have been performed with the same samples at 3 time points: freshly prepared hydrogels (day 0), after 1 day and after 3 days. The hydrogel samples were prepared in disks with a diameter of 5 mm and 2 mm height. Figure 7 shows the E-modulus at day 0, day 1 and day 3 and figure 8 shows the maximal force Fₘₐₓ at 60% stress at day 0, day 1 and day 3. Hydrogel H_{highHA} 310, 330 which contains more HA is stiffer than H_{lowHA} 320, 340. The E-modulus as well as the maximal force varies over time with the two materials behaving statistically significantly different over time for the E-modulus, but not statistically significantly different over time for the maximal force.

### Metabolic activity

Fig. 9 shows cell metabolic activity of the alginate-gelatin-HA hydrogel objects fabricated according to Example 2. Mesenchymal stem cells were mixed into the hydrogel at 37 °C at a density of 1 million cells/mL precursor mixture before printing. Samples were printed on the cooled substrate, cross-linked for 10 minutes in PBS with 2% (w/v) CaCl₂ and cultured in cell medium enhanced with 0.25% (w/v) CaCl₂ at 37 °C. Cell metabolic activity was measured 24 hours after printing measuring the fluorescence (F) of the alamarBlue^{®} assay and is shown normalized to hydrogel weight. Constructs without cells 350, 360 were compared to the constructs with cells 370, 380. Printed cells within the hydrogel objects 370, 380 survived the printing process and proved to be viable 24 hours after printing. Compared to the hydrogel objects without cells 350, 360, significantly higher cell viability was detected in the hydrogel objects with cells 370, 380. No statistical difference of cell viability between the two different hydrogel objects 370, 380 was observed. Unseeded hydrogels objects 350, 360 showed no metabolic activity.

Fig. 10 shows a LIVE/DEAD assay image of the alginate-gelatin-HA hydrogel objects fabricated according to Example 2. A representative sample of the group supplemented with high HA is shown. Mesenchymal stem cells were mixed into the hydrogel at 37 °C at a density of 1 million cells/mL precursor mixture before printing. Samples were printed on the cooled substrate, cross-linked for 10 minutes in PBS with 2% (w/v) CaCl₂ and cultured in cell medium enhanced with 0.25% (w/v) CaCl₂ at 37 °C. The LIVE-DEAD assay was performed on samples three days after printing. The LIVE/DEAD viability/cytotoxicity assay stains live and dead cells differently which emit two different wavelengths using fluorescence microscopy. A two-signal fluorescence cell image may be captured of living cells 390 and dead cells 400. Living cells could be detected on both HA compositions (H_{highHA} and H_{lowHA}).

### Rheological properties

Figs. 11 and 12 show viscosity and shear stress properties of an alginate-gelatin-HA hydrogel object fabricated according to Example 2. Rheological properties were based on rotatory measurements. The measurement was performed using a rheometer with a cone-plate measuring geometry (diameter 35mm, cone to plate distance 0.105 mm, cone angle 2°). A sample of 425-430 mg hydrogel (400 µl) was placed in the gap between the rotating cone and the stationary flat plate. Rotatory tests were performed to obtain viscosity and shear stress dependent of shear rate, which ranged between 0.1 and 3000 s⁻¹. The time duration for the whole measurement was set 800 s (400 seconds per way). A representative sample of H_{highHA} 410 and H_{lowHA} 420 of viscosity dependent, as well as H_{highHA} 430 and H_{lowHA} 440 of shear stress dependent on the shear rate is shown. Both hydrogels, H_{highHA} and H_{lowHA} reveal the same behavior of viscosity dependent on the shear rate.

Figs. 13 and 14 show the gel point of an alginate-gelatin-HA hydrogel object fabricated according to Example 2. Rheological properties were based on oscillatory measurements. For a representative H_{highHA} sample the temperature dependence of the storage modulus (G' 450) and loss modulus (G" 460) were measured using the temperature ramp oscillation mode with a constant shear stress amplitude of 50 Pa, a frequency of 1 Hz, and a temperature ramp of 1 °C/min. The temperature was set to 50 °C at the beginning of the test. During 80 min the sample is cooled down to 10°C and reheated to 50 °C. Same measurement settings were applied for a H_{lowHA} sample with storage modulus (G' 470) and ) loss modulus (G" 480). The intersection of the storage modulus G' and the loss modulus G" during the cooling phase from 50 °C to 10 °C demonstrates the gel point. It is an estimation of the gelation temperature of a thermosensitive material. The average gel point for H_{highHA} measured at 28.3 ± 0.6 °C (n=3), and for H_{lowHA} at 27.3 ± 0.3 °C (n=3).

## Claims

1. A process for preparing three-dimensional hydrogel objects comprising living cells, the process comprising:
(a) providing a cell suspension comprising a first hydrogel precursor, which exhibits a transition into a gel state by a reduction in temperature, comprising a second hydrogel precursor, which is chemically cross-linkable by a non-cytotoxic cross-linking agent, and comprising living cells;
(b) depositing the cell suspension onto a substrate by printing or plotting;
(c) cooling the deposited cell suspension to a temperature below the gel point of the cell suspension to solidify the deposited cell suspension; and
(d) adding a cross-linking agent to the solidified cell suspension so as to induce chemical cross-linking of the second hydrogel precursor.

2. The process according to claim 1, wherein the substrate is kept at a temperature below the gel point of the cell suspension, so as to cool the cell suspension after deposition.

3. The process according to claim 1 or 2, wherein the cell suspension is heated to a temperature above the gel point of the cell suspension before the cell suspension is deposited onto the substrate.

4. The process according to any one of the preceding claims, further comprising:
(e) eluting the first hydrogel precursor after chemical cross-linking of the second hydrogel precursor to create a porous three-dimensional hydrogel object comprising living cells.

5. The process according to any one of the preceding claims, wherein the gel point of the cell suspension is between approximately 0 °C and approximately 25 °C.

6. The process according to any one of the preceding claims, wherein the first hydrogel precursor is at least one of gelatin and a mixture of pectin and chitosan.

7. The process according to any one of the preceding claims, wherein the second hydrogel precursor is at least one of alginate, cellulose-derivatives such as sodium carboxymethylcellulose or hydroxyethyl cellulose, chitosan, chondroitin sulphate, chondroitin sulfate-PEG, hyaluronic acid, pectin gel, poly(L-lactide), poly(vinyl alcohol), xanthan, and fibrin.

8. A composition for preparing three-dimensional hydrogel objects comprising:
a first hydrogel precursor, which exhibits a transition into a gel state by a reduction in temperature;
a second hydrogel precursor, which is chemically cross-linkable by a non-cytotoxic cross-linking agent; and
living cells.

9. The composition according to claim 8, further comprising an X-ray opaque material.

10. The composition according to claim 8 or 9, wherein the gel point of the composition is between approximately 0 °C and approximately 25 °C.

11. The composition according to any one of claims 8-10, wherein the first hydrogel precursor is at least one of gelatin and a mixture of pectin and chitosan.

12. The composition according to any of claims 8-11, wherein the second hydrogel precursor is at least one of alginate, cellulose derivatives such as sodium carboxymethylcellulose or hydroxyethyl cellulose, chitosan, chondroitin sulphate, chondroitin sulfate-PEG, hyaluronic acid, pectin gel, poly(L-lactide), poly(vinyl alcohol), xanthan, and fibrin.

13. Use of a composition according to any one of claims 8-12 for printing or plotting three-dimensional hydrogel objects comprising living cells.

14. An apparatus for preparing three-dimensional hydrogel objects comprising living cells, the apparatus comprising:
(a) a discharge device for discharging a cell suspension onto a substrate;
(b) a heating device in contact with said discharge device for heating the cell suspension before discharge;
(c) an X-Y positioning platform for positioning the discharge device and the substrate relative to each other in a predetermined plane;
(d) a cooling device for cooling the cell suspension after discharge; and
(e) a Z positioning platform for positioning the discharge device and the substrate relative to each other in a direction perpendicular to the substrate.
